# EUROPEAN PATENT APPLICATION

(11) **EP 2 181 980 A1**
(43) Date of publication of application: **05.05.2010**
(21) Application number: 08382059.7
(22) Date of filing: 28.10.2008
(51) Int. Cl.: C07C 211/42, C07C 271/24, C07C 271/36

(54) **A process for the preparation of (R)-1-aminoindanes**

(71) Applicant: Chemo Ibérica, S.A., 08028 Barcelona (ES)
(72) Inventor: Marras, Giovanni, 28100 Novara (Novara) (IT); Rasparini, Marcello, 27010 Cura Carpignano (PV) (IT); Tufaro, Roberto, 28060 Sozzago (Novara) (IT); Castaldi, Graziano, 28072 Briona (NO) (IT); Ghisleri, Diego, 27050 Casei Gerola (PV) (IT)
(74) Representative: ZBM Patents

(57) **Abstract**

The present invention relates to a process for the preparation of the chiral compound of formula (V), wherein R₁ is a C₁-C₆ alkyl group optionally substituted with halogens, an aryl optionally substituted, or a trialkyl silyl group, for use as an intermediate in the preparation of (*R*)-1-amino indanes, helpful in the pharmaceutical field. The compound of formula (V) is a key intermediate to the preparation of (*R*)-1-aminoindanes, particularly Rasagiline. The invention also relates to novel compounds, which are especially useful as intermediates for the preparation of (*R*)-1-aminoindanes.

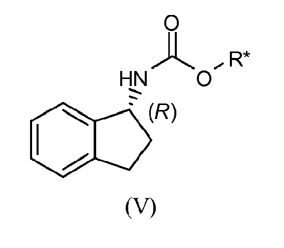

## Description

### Field of the invention

The present invention relates to a process for the preparation of (R)-1-aminoindanes, particularly Rasagiline, and intermediates for the synthesis thereof.

### Background of the invention

(*R*)-1-aminoindanes are useful as intermediates for the preparation of pharmaceutical compounds for the treatment of various CNS disorders, as described in EP-738149-A. In particular, the (*R*)-1-aminoindane Rasagiline is a potent, selective irreversible inhibitor of the B-form of the monoamine oxidase enzyme (MAO-B), responsible for the breakdown of dopamine after its release into the synapse. By inhibiting MAO-B, Rasagiline prevents the deamination of the monoamines dopamine and phenethylamine, thereby increasing their concentration in the synapse. Rasagiline is particularly useful for the treatment of Parkinson's disease, that is widely considered to be the result of degradation of the pre-synaptic dopaminergic neurons in the brain, with a subsequent decrease in the amount of the neurotransmitter dopamine, that is being released. Rasagiline both raises levels of striatal dopamine produced from levodopa and improves the survival prospects of ailing dopaminergic neurons themselves. Rasagiline is also suitable for treating memory disorders and dementia of the Alzheimer type, depression, and hyperactive syndrome in children. The main therapeutic advantage of Rasagiline over other MAO inhibitors is that it doesn't have the presumed toxic metabolic amphetamine breakdown products, in respect with the structurally similar Selegiline.

Rasagiline is a compound of formula (I) 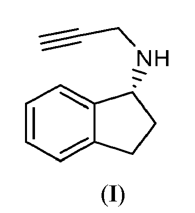 chemically known as (*R*)-(+)-*N*-propargyl-1-aminoindan, which is disclosed in EP 436 492 A and marketed under the trade name Azilect^{®}.

(*S*)- and (*R*)- enantiomers of 1-aminoindan are commercially available, but are expensive.

Several methods have been used to prepare 1-aminoindanes or Rasagiline.
Patent application EP 235 590 describes a process of resolving 1-aminoindanes into the R-isomer, by using (*R*)-N-acetyl-3,4-dimethoxyphenylalanine as the resolving agent.
Patent application EP 436 492 A described a process to obtain Rasagiline, comprising the alkylation of the racemic 1-aminoindan (X) 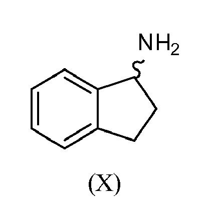 with propargyl bromide and subsequently optical resolution to Rasagiline.
Patent application WO 2006120577 relates to a method for the preparation of Rasagiline by converting the hydroxyl group of (*S*)-(-)-1-indanol to a leaving group and subsequently reacting with propargylamine to give Rasagiline.
Patent application WO 2002068376 relates to a method for the preparation of Rasagiline by reacting racemic *N*-benzyl-1-indamine with (*R*,*R*)-tartaric acid to obtain (*R*)-1-aminoindan, and subsequently conversion to Rasagiline.

The active substance prepared by the processes known up till now can only be obtained in a satisfactory quality after running through a number of process steps. There is therefore the need for an alternative synthesis for the industrial preparation of (*R*)-1-aminoindanes, particularly Rasagiline, which makes use of commercially available, cheaper or easy to prepare intermediates.

An object of the present invention is to provide alternative, efficient, economic and commercially useful processes for the manufacture of (*R*)-1-aminoindanes, particularly Rasagiline, that avoids the above-identified problems. The compound of formula (V) is a key intermediate to the preparation of (*R*)-1-aminoindanes. The invention also relates to novel compounds, which are especially useful as intermediates for the preparation of (*R*)-1-aminoindanes.

### Summary of the invention

It has now been found a process for the preparation of (*R*)-1-aminoindanes in a straightforward manner exploiting novel intermediates.

The method of the present invention involves a process for preparing a compound of formula (V), 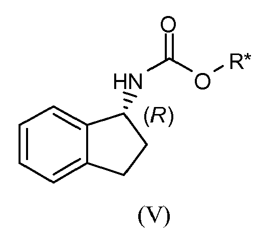 with the (*R*)- configuration at the 1-position of indane ring; wherein R* is a chiral moiety having at least one asymmetric center; the process comprising:
a) reacting a compound of formula (II), 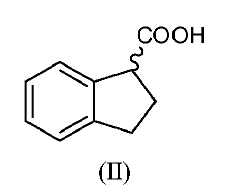 with a reagent capable of transforming a compound of formula (II) into an isocyanate of formula (III), 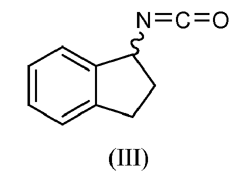 and,
b) reacting isocyanate of formula (III) with a chiral alcohol R*-OH, wherein R* is a chiral moiety having at least one asymmetric center to obtain a carbamate of formula (IV), as a mixture of diastereoisomers 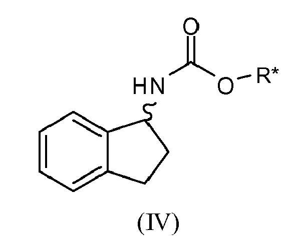 wherein R* is as defined above;
c) resolving the mixture of diastereoisomers of carbamates of formula (IV) to obtain a diastereoisomer of formula (V), 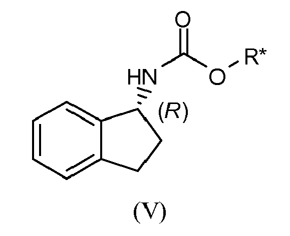 wherein R* is as defined above, with the (*R*)- configuration at the 1-position of indane ring.

In another aspect, the invention provides a process for the preparation of Rasagiline (I), or a pharmaceutically acceptable salt, hydrate, solvate, or polymorph thereof, said process comprising carrying out the preparation process of compound V according to the process mentioned above.

According to the invention, compounds of formula (V) may also be prepared by reaction of the racemic 1-aminoindane (X) with a chloroformate of formula (XI) 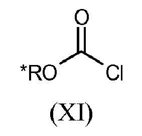 wherein R* is defined as above, in an inert solvent, such as a polar aprotic solvent, for example dichloromethane, tetrahydrofuran; and in the presence of a base, for instance a tertiary amine.

As said before, the compound of formula (V) is a key intermediate to the preparation of (*R*)-1-aminoindanes.

In another aspect, the invention provides a process for the preparation of Rasagiline (I), or a pharmaceutically acceptable salt, hydrate, solvate, or polymorph thereof, by:
a) transterification of carbamate of formula (V) into a carbamate of formula (VI) 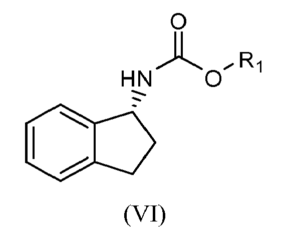 wherein R₁ is a C₁-C₆ alkyl group optionally substituted with halogens, an aryl optionally substituted, or a trialkyl silyl group,
b) reacting a compound of formula (VI), as defined above, with a compound of formula (VII), 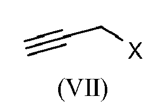 wherein X is a leaving group; to obtain a compound of formula (VIII) 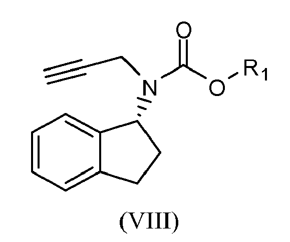 wherein R₁ is as defined above; and
f) deprotecting a compound of formula (VIII), to give Rasagiline (I).

In another aspect, the invention provides a process for the preparation of Rasagiline (I), or a pharmaceutically acceptable salt, hydrate, solvate, or polymorph thereof, by:
a) converting the compound of formula (V), as defined above, to an (*R*)-aminoindan of formula (IX) 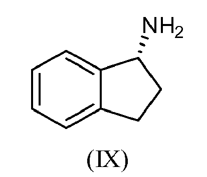 or a pharmaceutically acceptable salt, hydrate, solvate, or polymorph thereof,
b) reacting (*R*)-aminoindan of formula (IX) with a dihalopropene compound of formula (XII): 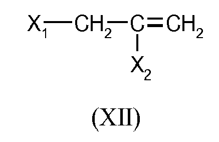 where X₁ and X₂ independently represent a chlorine, bromine or iodine to obtain a haloallyl derivative of formula (XIII): 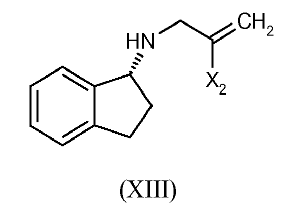 or a pharmaceutically acceptable salt, hydrate, solvate or polymorph thereof wherein X₂ is as defined above
c) dehydrohalogenation of compound of formula (XIII) with a base to obtain rasagiline of formula (I).

In another aspect, the invention provides a process for the preparation of Rasagiline (I), or a pharmaceutically acceptable salt, hydrate, solvate, or polymorph thereof, by:
a) reacting racemic 1-aminoindane of Formula (X) 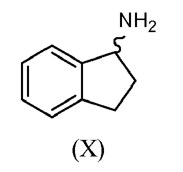 with a dihalopropene of formula (XII) as defined above to afford a compound of formula (XIV) 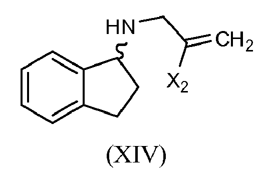 or a pharmaceutically acceptable salt, hydrate, solvate or polymorph thereof, wherein X₂ is as defined above
b) resolving the mixture of diastereoisomers of formula (XIV) to obtain a diastereoisomer of formula (XIII) 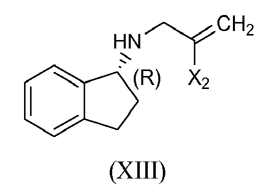 or a pharmaceutically acceptable salt, hydrate, solvate or polymorph thereof; wherein X₂ is as defined above, with the (R)-configuration at the 1-position of the indane ring
c) dehydrohalogenation of compound of formula (XIII) with a base to obtain rasagiline of formula (I).

In another aspect, the invention provides a process for the preparation a (*R*)-aminoindan of formula (IX) 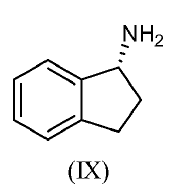 or a pharmaceutically acceptable salt, hydrate, solvate, or polymorph thereof, by hydrolysing a compound of formula (V) or (VI) as defined above.

In another aspect, the invention provides a compound of Formula (IV), as defined above, or a pharmaceutically acceptable salt, hydrate, solvate, or polymorph thereof.

In another aspect, the invention provides a compound of Formula (V), as defined above, or a pharmaceutically acceptable salt, hydrate, solvate, or polymorph thereof.

In another aspect, the invention provides a compound of Formula (VI), as defined above, or a hydrate, solvate, or polymorph thereof, with the proviso that R1 is not a t-butyl group.

In another aspect, the invention provides a compound of Formula (VIII), as defined above, or a pharmaceutically acceptable salt, hydrate, solvate, or polymorph thereof.

In another aspect, the invention provides a compound of Formula (XIII), as defined above, or a pharmaceutically acceptable salt, hydrate, solvate or polymorph thereof.

In another aspect, the invention provides a compound of Formula (XIV), as defined above, or a pharmaceutically acceptable salt, hydrate, solvate or polymorph thereof.

In another aspect, the invention provides the use of compounds of formula (IV), (V) or (VI), as defined above, as intermediates in the preparation of (*R*)-1-aminoindanes.

In another aspect, the invention provides the use of compounds of formula (IV), (V), (VI), (VIII), (XIII), or (XIV) as defined above, as intermediates in the preparation of (*R*)-1-aminoindanes, preferably Rasagiline.

### Brief description of the Figures

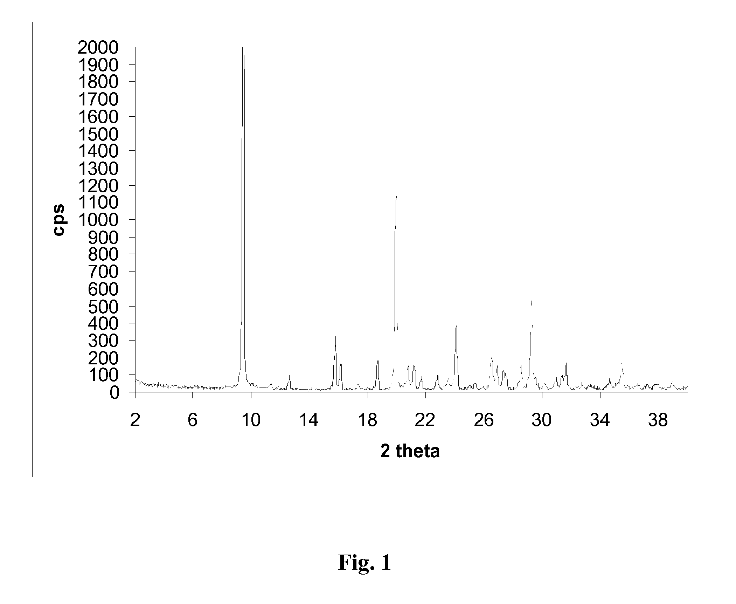

Fig. 1: PXRD of (*R*)-*N*-(2-chloroallyl)-2,3-dihydro-1H-inden-1-amine oxalate (XIIIb)

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The terms "alkyl", "C₁-C₆ alkyl" refer to a straight or branched hydrocarbon having from 1 to 6 carbon atoms. Examples of alkyl groups include, without limitation, methyl, ethyl, *n*-propyl, isopropyl, *n*-butyl, *sec*-butyl, isobutyl, *tert*-butyl, *n*-pentyl, *n*-hexyl, and the like. A preferred C₁-C₆ alkyl group of the present invention is isopropyl or *tert*-butyl, more preferably *tert*-butyl.

The term "C₁-C₈ alkyl" refers to a straight or branched hydrocarbon having from 1 to 8 carbon atoms, hence comprehensive of the aforementioned "C₁-C₆ alkyl" and also comprising heptyl, octyl, and the like.

The term "aryl" refers to an aromatic carbocyclic group having a single ring (e.g., phenyl), multiple rings (e. g., biphenyl), or multiple condensed rings in which at least one is aromatic (e.g., 1,2,3,4-tetrahydronaphthyl, 1-naphthyl, 2-naphthyl, anthryl, phenanthryl or fluorenyl). The aryl group may be optionally substituted. A preferable aryl group of the present invention is phenyl.

The term "halogen" refers to chlorine, bromine, fluoride or iodine.

Unless otherwise stated, the term "substituted" refers to groups substituted with from 1 to 5 substituents selected from the group consisting of C₁-C₈ alkyl, halogen, aryl, and the like.

The term "leaving group" refers preferably one of the groups chloride, bromide, iodide, mesylate, tosylate, triflate; preferably bromide or chloride, more and more preferably bromide.

The term "racemic" refers to a sample of a chiral compound which contains both the (+) and (-) isomers in equal amounts.

The term "about" encompasses the range of experimental error that may typically occur in a measurement.

The wavy bond refers to both (*R*) and (*S*) configuration at the C-N bond of the indan ring.

The terms "(*R*)-1-aminoindanes", "(*R*)-Rasagiline" refer to enantiomerically pure (*R*)-1-aminoindanes, (*R*)-Rasagiline, or enantiomerically enriched (*R*)-1-aminoindanes, (*R*)-Rasagiline.

The term "enantiomerically enriched" refers to a sample of two enantiomers having an enantiomeric ratio (*i.e.* the ratio between the two enantiomers) of, for example, ≥80: ≤ 0, preferably >90 : ≤ 10, more preferably ≥95 : ≤ 5.

The term "enantiomerically pure" refers to a sample containing greater than about 95% of the desired enantiomer, preferably greater than about 98% of the desired enantiomer, and more preferably greater than about 99.5% of the desired enantiomer. For example, the enantiomerically pure (*R*)-Rasagiline is substantially free of (*S*)-Rasagiline. The term "substantially free" means that a diastereoisomer or enantiomer contains less than about 5%, preferably less than about 2%, and more preferably less than about 0.5% of the other enantiomer.

The term "diastereomerically enriched" refers to a sample of two diastereoisomers having an diastereomeric ratio (*i.e.* the ratio between the two diastereoisomers) of, for example, ≥80: ≤ 20, preferably ≥90 : ≤ 10, more preferably ≥95 : ≤ 5.

The term "diastereomerically pure" refers to a sample containing greater than about 95% of the desired diastereoisomer, preferably greater than about 98% of the desired diastereoisomer, and more preferably greater than about 99.5% of the desired diastereoisomer.

The term "pharmaceutically acceptable salt(s)", as used herein, unless otherwise indicated, includes salts of acidic or basic groups which may be present in the compounds of the present invention. The compounds prepared according to the present invention that are basic in nature are capable of forming a wide variety of salts with various inorganic and organic acids. The acids that may be used to prepare pharmaceutically acceptable acid addition salts of such basic compounds of are those that form non-toxic acid addition salts i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, oxalate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds prepared according to the present invention that include a basic moiety, such as an amino group, may form pharmaceutically acceptable salts with various amino acids, in addition to the acids mentioned above.

The term "hydrate" refers to a solvate comprising a disclosed or claimed compound and a stoichiometric or non-stoichiometric amount of water.

The term "solvate" refers to a molecular complex comprising a disclosed or claimed compound and a stoichiometric or non-stoichiometric amount of one or more solvent molecules (e.g., ethanol).

The term "polymorph" refers to substances that have the same chemical formula, but different crystal structures

The term "one pot" process refers to a series of consecutive reactions which are carried out without isolating the respective intermediates.

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.

The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

The processes of the invention are illustrated in Scheme 1. 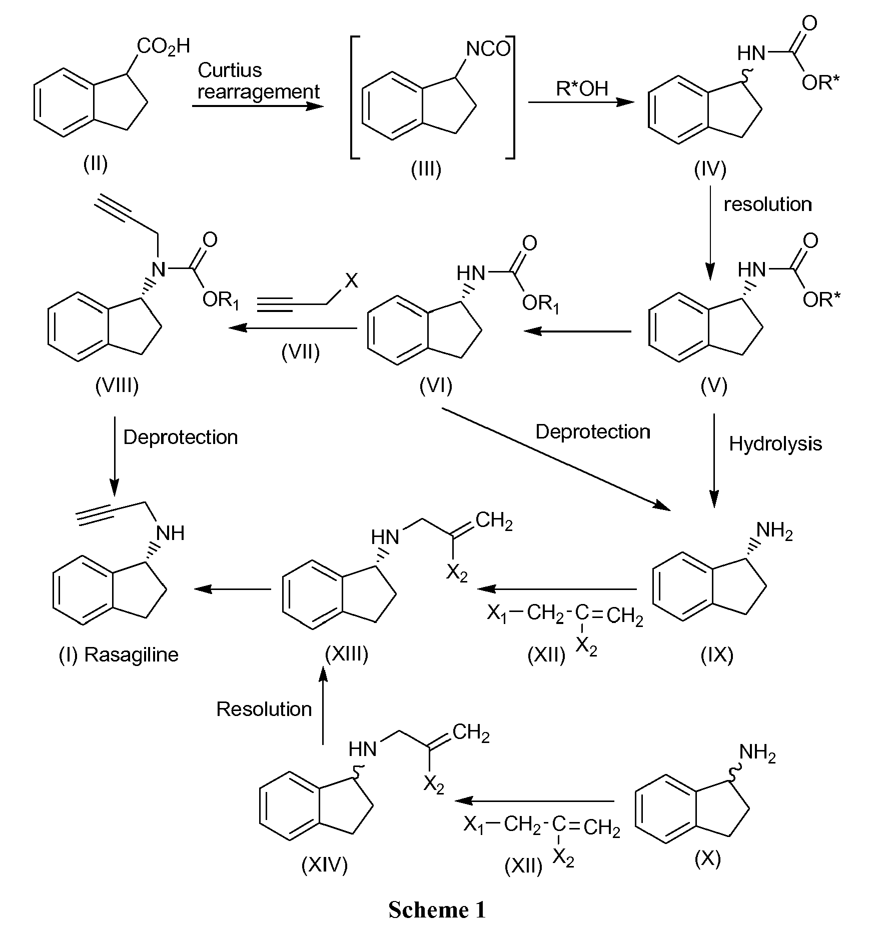 wherein R*, R₁ and X are as defined above.

In a first aspect, the present invention provides a method for obtaining intermediate of formula (V), 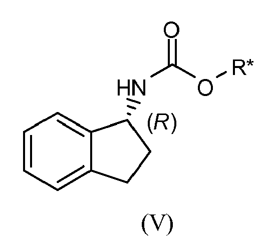 with the (*R*)- configuration at the 1-position of indane ring; wherein R* is a chiral moiety having at least one asymmetric center; the process comprising:
a) reacting a compound of formula (II), 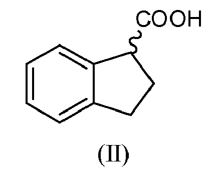 with a reagent capable of transforming a compound of formula (II) into an isocyanate of formula (III), 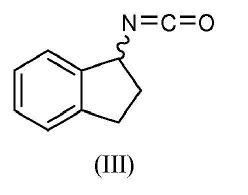 and,
b) reacting isocyanate of formula (III) with a chiral alcohol R*-OH, wherein R* is a chiral moiety having at least one asymmetric center to obtain a carbamate of formula (IV), as a mixture of diastereoisomers, 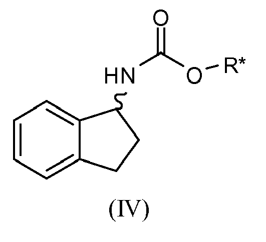 wherein R* is as defined above;
c) resolving the mixture of diastereoisomers of carbamates of formula (IV) to obtain a diastereoisomer of formula (V), 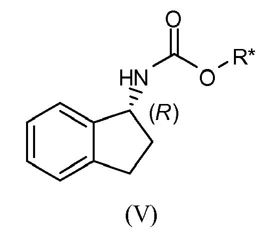 wherein R* is as defined above, with the (*R*)- configuration at the 1-position of indane ring;

Preferably, step b) is carried out in the presence of a basic agent.

Known compound of formula (II) may be prepared from indene, a cheap industrial starting material, as described in Cromwell N.H., Caps D.B., Journal of the American Chemical Society 1952, 4448-4449.

In a preferred embodiment, compound of formula (II) is transformed into a isocyanate of formula (III) *via* a Curtius rearrangement, for example by reaction with diphenylphosphoryl azide (DPPA), preferably in the presence of an inert solvent, for instance a non polar solvent, such as an aromatic hydrocarbon, such us toluene or xylene, preferably toluene; or a polar aprotic solvent, such as tetrahydrofuran or dichloromethane.

Preferably, the reaction is carried out at a temperature comprised from room temperature to the reflux temperature of the reaction mixture, preferably between about 60 and 80 °C.

Advantageously, DPPA is employed in a molar ratio between 1.0 and 2.0 to the acid (II), preferably in a molar ratio between 1.0 and 1.2.

In a preferred embodiment, stages a) and b) are carried out in a "one pot" reaction.

Thus, according to said "one pot" reaction isocyanate (III) is not isolated and is directly trapped in the reaction mixture with an alcohol R^{*}-OH, wherein R* is a chiral moiety having at least one asymmetric center, preferably in the presence of a basic agent to give carbamate (IV), as a mixture of diastereoisomers. Preferably, R* is selected from (-)-menthyl, (+)-menthyl, (-)-8-phenylmenthyl, (+)-8-phenylmenthyl, (-)-neomenthyl, (+)-neomenthyl and (R) and (S)-1-phenylethyl.

Examples of preferred alcohols R^{*}-OH include but are not limited to (-)-menthol, (+)-menthol, (-)-8-phenylmenthol, (+)-8-phenylmenthol, (-)-neomenthol, (+)-neomenthol, (*R*) or (*S*)-1-phenylethanol, more preferably (-)-menthol or (+)-menthol, more preferably (-)-menthol.

Preferably, the preferred alcohol R^{*}-OH is employed in a molar ratio of 1.0 to 2.0 to the acid (II), more preferably in a molar ratio between 1.0 and 1.2.

Examples of suitable basic agents include but are not limited to organic bases, for instance a tertiary amine, such as triethylamine, tributylamine, ethyldiisopropylamine or *N*-methylmorpholine, preferably triethylamine. Preferably, tertiary amine is employed in a molar ratio of 1.0 to 2.0 to the acid (II), preferably in a molar ratio from 1.0 to 1.5.

Preferably, the reaction is carried out at a temperature comprised from room temperature to the reflux temperature of the reaction mixture, preferably between about 60 and 80 °C.

In another preferred embodiment of the invention, the diastereoisomeric mixture of carbamates of formula (IV) is resolved by fractional crystallization to obtain the desired diastereoisomer of formula (V) by dissolution in a solvent, preferably an alcohol, such as methanol, ethanol, *iso*-propanol, *n*-propanol, preferably methanol, and crystallizating at a temperature comprised from about 40 °C to 60 °C, preferably at a temperature between about 45 °C and 55 °C, and isolating the desired diastereoisomer. If necessary the process may be repeated to enhance the diastereoisomeric purity of the product.

Compounds of formula (IV) and compounds of formula (V) are novel compounds and are also object of the present invention.

Preferred compounds of formula (IV) or (V) are compounds wherein R* is selected from (-)-menthyl, (+)-menthyl, (-)-8-phenylmenthyl, (+)-8-phenylmenthyl, (-)-neomenthyl, (+)-neomenthyl, and (*R*) or (*S*)-1-phenylethyl, preferably (-)-menthyl and (+)-menthyl, more preferably (-)-menthyl.

Preferred examples of compounds of formula (IV) or (V) are:
(1R,2S,SR)-2-isopropyl-5-methylcyclohexyl (*R*)-2,3-dihydro-1H-inden-1-ylcarbamate;
(1R,2S,5R)-2-isopropyl-5-methylcyclohexyl (*S*)-2,3-dihydro-1H-inden-1-ylcarbamate;
(1*S*,2*R*,5*S*)-2-isopropyl-5-methylcyclohexyl (*R*)-2,3-dihydro-1H-inden-1-ylcarbamate;
(1*S*,2*R*,5*S*)-2-isopropyl-5-methylcyclohexyl (*S*)-2,3-dihydro-1H-inden-1-ylcarbamate.

In another aspect, the invention provides a process for the preparation of Rasagiline (I), or a pharmaceutically acceptable salt, hydrate, solvate, or polymorph thereof, said process comprising carrying out the preparation process of compound V according to the process mentione above.

In a further aspect, the present invention provides a method for obtaining intermediate of formula (V) comprising reacting racemic 1-aminoindane (X) with a chloroformate of formula (XI), 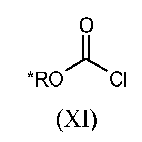 wherein R* is defined as above.

In a preferred embodiment, the reaction is carried out in an inert solvent, such as a polar aprotic solvent, for example dichloromethane, tetrahydrofuran.

Preferably the reaction is conducted in the presence of a base. Examples of suitable bases include but are not limited to inorganic bases, such as sodium hydroxide, potassium hydroxide, sodium methoxide, sodium ethoxide, potassium t-butoxide or organic bases, such as pyridine, triethylamine, diisopropylamine, 1,8-diazabicyclo[5,4,0]undec-7-ene, and ethylendiamine.

Another object of the present invention is to provide a process for the preparation of Rasagiline (I), or a pharmaceutically acceptable salt, hydrate, solvate, or polymorph thereof, 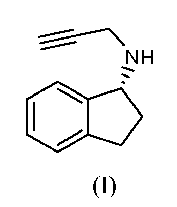 said process comprising:
a) transterification of carbamate (V) to a more labile carbamate (VI), in order to release the amine nitrogen under milder conditions compared to those necessary to deprotect carbamate (V) 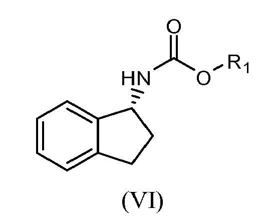 wherein R₁ is a C₁-C₆ alkyl group optionally substituted with halogens, an aryl optionally substituted, or a trialkyl silyl group,
b) reacting a compound of formula (VI) , with a compound of formula (VII) 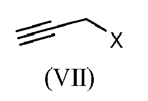 wherein X is a leaving group, such as chloride, bromide, iodide, mesylate, tosylate, triflate, preferably propargyl bromide or chloride, more preferably propargyl bromide; to obtain a compound of formula (VIII) 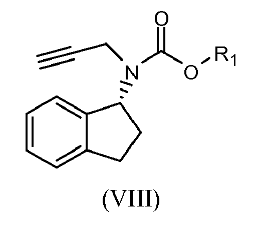 and,
c) deprotecting a compound of formula (VIII), to give Rasagiline (I).

Preferably, R* is selected from (-)-menthyl, (+)-menthyl, (-)-8-phenylmenthyl, (+)-8-phenylmenthyl, (-)-neomenthyl, (+)-neomenthyl or (R) or (S)-1-phenylethyl.

Also preferably, R₁ is selected from tetra-*is*opropyl, tetra-*tert*-butyl, tetra 2,2,2-trichloroethyl, tetrabenzyl and tetra (2-trimethylsilylethyl).

In a preferred embodiment, the carbamate of formula (V) is transesterified into carbamate of formula (VI) by reaction with an orthotitanate Ti(OR₁)₄, wherein R₁ is as defined above. Preferably, Ti(OR₁)₄ is selected from tetra-*iso*propyl orthotitanate, tetra-*tert*-butyl orthotitanate, tetra 2,2,2-trichloroethyl orthotitanate, tetrabenzyl orthotitanate, tetra (2-trimethylsilylethyl) orthotitanate, more preferably tetra-*tert*-butyl orthotitanate.

Suitably, the transterification reaction is carried out employing a molar ratio of tetraalkyl orthotitanate to carbamate of formula (V) comprised from 1 to 3, preferably a molar ratio from 1 to 1.5.

Preferably, the reaction is carried out in an inert solvent, such as an aromatic hydrocarbon, for example toluene or xylenes, optionally using an alcohol R₁OH as co-solvent, wherein R₁ is as defined above, at a temperature comprised from about 50 °C to the reflux temperature of the mixture, preferably at reflux temperature.

Compounds of formula (VI) with the proviso that R₁ is not a t-butyl group, are novel compounds and are also object of the present invention. Preferred examples of formula (VI) are:
(*R*)-isopropyl 2,3-dihydro-1H-inden-1-ylcarbamate;
(*R*)-2,2,2-trichloroethyl 2,3-dihydro-1H-inden-1-ylcarbamate.

According to a preferred embodiment the reaction for the preparation of compound of formula (VIII) is carried out in the presence of a basic agent, such as sodium, potassium or lithium hydroxide, sodium or potassium hydride, sodium or potassium *tert*-butoxide, sodium amide; or in the presence of an alkyllithium, such as *n*-butyllithium or *n*-hexyllithium; or in the presence of an alkyl magnesium halide, such as ethylmagnesium bromide or chloride, *iso*propylmagnesium bromide or chloride; or in the presence of a phase transfer catalyst, such as tetrabutylammonium bromide, tetrabutylammonium chloride, tetrabutylammonium iodide or tetrabutylammonium hydrogensulfate, preferably tetrabutylammonium hydrogensulfate

Preferably, the reaction for the preparation of compound of formula (VIII) is carried out in the presence of a basic agent. Preferred basic agent is potassium hydroxide or sodium hydride.

Preferably, the reaction for the preparation of compound of formula (VIII) is carried out in a polar aprotic solvent, such as dimethylformamide, dimethylacetamide, *N*-methylpyrrolidone, tetrahydrofuran, dioxane or mixtures thereof; or a non polar solvent, such as toluene, xylenes or mixtures thereof. Preferred solvents are toluene and tetrahydrofuran.

Preferably, the base and/or the compound of formula (VII) are employed in a molar ratio to carbamate of formula (VI) comprised from about 1.0 to 2.0, preferably from about 1.2 to 1.5.

The reaction is carried out at a temperature comprised between about -15 and 50 °C, preferably at approximately 50°C.

Compounds of formula (VIII) are novel compounds and are also object of the present invention. Preferred examples of compound (VIII) are:
(*R*)-*tert*-butyl 2,3-dihydro-1H-inden-1-yl(prop-2-ynyl)carbamate;
(*R*)-isopropyl 2,3-dihydro-1H-inden-1-yl(prop-2-ynyl)carbamate;
(*R*)-2,2,2-trichloroethyl 2,3-dihydro-1H-inden-1-yl(prop-2-ynyl)carbamate.

Deprotection reaction of compound of formula (VIII) can be carried out as described for example in Greene T.W., Wuts P.G.M., "Protective groups in organic synthesis", 3rd ed., 1999

The type of the deprotection reaction to be carried out can be selected depending on the nature of R₁. The deprotection can be a hydrolysis. Exemplary deprotection reactions include:
a) an alkaline hydrolysis employing a base, inorganic or organic base, such as lithium, sodium, potassium hydroxide, or the like; aliphatic amines, heterocyclic amines, such as piperidine, or the like, preferably potassium hydroxide, in a solvent, such as water or an alcohol, such as methanol, ethanol or 1-butanol or 2-butanol, or an ether, such as tetrahydrofuran, dichloromethane or mixtures thereof;
b) an acidic treatment in a suitable solvent. The acid can be a suitable organic or inorganic acid. A suitable organic acid is, for example, acetic acid, trifluoroacetic acid, formic acid, methanesulfonic or p-toluenesulfonic acid, preferably trifluoroacetic acid or methanesulfonic acid. A suitable inorganic acid is hydrochloric acid or sulfuric acid, preferably hydrochloric acid. A suitable solvent is an inert solvent, such as dichloromethane, or an ether, such as tetrahydrofuran or dioxane, preferably dioxane, or an alcohol, such as methanol or isopropanol, preferably isopropanol;
c) action of a nucleophilic agent. The nature of the nucleophile can be determined according to methods well known to those skilled in the art, depending on the nature of the group to deprotect. For instance, the nucleophile can be a thiolate, a fluoride anion, an hydride, such as lithium borotriethylhydride, hydrogen.

Another object of the present invention is to provide a process for the preparation of Rasagiline (I), or a pharmaceutically acceptable salt, hydrate, solvate, or polymorph thereof, 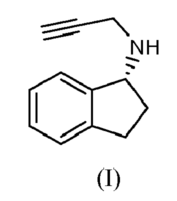 said process comprising
a) converting the compound of formula (V), as defined above, to an (*R*)-aminoindan of formula (IX) 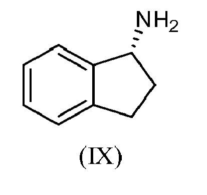
b) reacting (*R*)-aminoindan of formula (IX) with a dihalopropene compound of formula (XII) 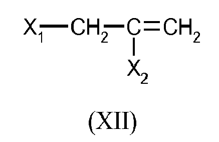 where X₁ and X₂ independently represent chlorine, bromine or iodine, to obtain a haloallyl derivative of formula (XIII) 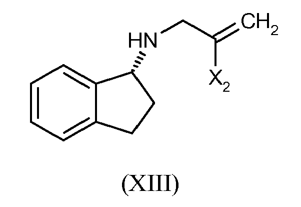 wherein X₂ is as defined above, and
c) dehydrohalogenating compound of formula (XIII) with a base to obtain rasagiline of formula (I).

According to a preferred embodiment, carbamates of formula (V) are treated with an inorganic or organic acid, such as hydrochloric acid, trifluoroacetic acid; or with an inorganic base, such as sodium or potassium hydroxide, piperidine; or with a nucleophilic agent. The nature of the nucleophile can be determined according to methods well known to those skilled in the art, depending on the nature of the group to deprotect. For instance, the nucleophile can be a thiolate, a fluoride anion, an hydride, such as lithium borotriethylhydride, hydrogen.

Preferably, the hydrolysis of the compounds of formula (V) to the (*R*)-1-aminoindane (IX), is conducted in a high boiling alcoholic solvent, such as 1- or 2-butanol, followed by extraction and purification of the desired amine with the ordinary methods known to those skilled in the art.

Examples of compound (XII) include 2,3-dichloro-1-propene, 2,3-dibromo-1-propene, 2,3diiodo-1-propene, 2-chloro-3-bromo-1-propene, 2-chloro-3-iodo-1-propene, 2-bromo-3-chloro-1-propene, 2-bromo-3-iodo-1-propene, 2-iodo-3-chloro-1-propene, and 2-iodo-3-bromo-1-propene. Preferred examples of compound (XII) are 2,3-dichloro-1-propene and 2,3-dibromo-1-propene, more preferred 2,3-dichloro-1-propene.

Preferably, the compound of formula (XII) is employed in a molar ratio to (*R*)-aminoindan of formula (IX) comprised from about 1.0 to 10.0, preferably from 1.0 to 2.0, more preferably from 1.0 to 1.5.

According to a preferred embodiment the reaction for the preparation of compound of formula (XIII) is carried out in the presence of a base. Examples of base include but are not limited to sodium carbonate, potassium carbonate, sodium bicarbonate, potassium bicarbonate, sodium hydroxide, potassium hydroxide, etc. Preferred bases are sodium carbonate and potassium carbonate, more preferred potassium carbonate.

According to another preferred embodiment, the reaction is carried out in presence of an activating agent, such as an alkali metal halide. Preferred alkali metal halides are sodium iodide and potassium iodide, more preferably potassium iodide.

Preferably, the reaction for the preparation of compound of formula (XIII) is carried out in a polar aprotic solvent, such as acetonitrile, dimethylformamide, dimethylacetamide, *N*-methylpyrrolidone, dimethylsulfoxide, tetrahydrofuran, dioxane or mixtures thereof. Preferred solvent is acetonitrile.

The reaction is carried out at a temperature comprised between about 0° to 100 °C, preferably 20 to 60°C.

The compounds of Formula (XIII) can be obtained as crude or pure products, which can optionally be converted into pharmaceutically acceptable salts, using methods well known to those skilled in the art. Upon basic treatment, diastereomerically enriched salts can liberate enantiomerically pure haloallyl compounds of Formula (XIII).

Compounds of formula (XIII) and their pharmaceutically acceptable salts are novel compounds and are also object of the present invention. Preferred examples of compound (XIII) are:
(*R*)-*N*-(2-chloroallyl)-2,3-dihydro-1H-inden-1-amine
(*R*)-*N*-(2-bromoallyl)-2,3-dihydro-1H-inden-1-amine
(*R*)-*N*-(2-chloroallyl)-2,3-dihydro-1H-inden-1-amine oxalate

The dehydrohalogenation reaction of compound of formula (XIII) is conducted in the presence of a base. Examples of suitable bases include but are not limited to inorganic bases, such as sodium hydroxide, potassium hydroxide, sodium methoxide, sodium ethoxide, potassium t-butoxide or organic bases, such us pyridine, triethylamine, diisopropylamine, 1,8-diazabicyclo[5,4,0]undec-7-ene, and ethylendiamine.

The amount of base employed is usually 1 to 10 parts by mole, preferably 1 to 4 parts by mole, based on the haloallyl derivative of formula (XIII).

Preferably, the dehydrohalogenation reaction is carried out in a polar aprotic solvent, such as acetonitrile, dimethylformamide, dimethylacetamide, N-methylpyrrolidone, dimethylsulfoxide, tetrahydrofuran, dioxane or mixtures thereof. Preferred solvent is dimethylsulfoxide.

The reaction is carried out at a temperature comprised between about 0° to 100 °C, preferably 20 to 60°C.

Another object of the present invention is to provide a process for the preparation of Rasagiline (I), or a pharmaceutically acceptable salt, hydrate, solvate, or polymorph thereof, 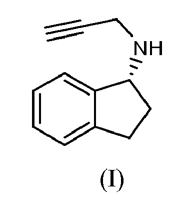 said process comprising
a) reacting racemic 1-aminoindane of Formula (X) 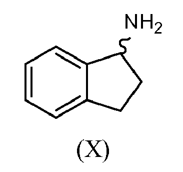 with a dihalopropene of formula (XII) as defined above to afford a compound of formula (XIV) 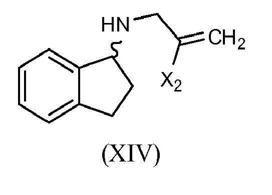 or a pharmaceutically acceptable salt, hydrate, solvate or polymorph thereof;
   wherein X₂ is as defined above
b) resolving the mixture of diastereoisomers of formula (XIV) with a chiral acid to obtain a compound of formula (XIII), substantially free of the other enantiomer 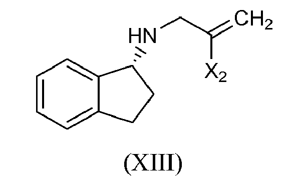 or a pharmaceutically acceptable salt, hydrate, solvate or polymorph thereof, wherein X₂ is as defined above
c) dehydrohalogenation of compound of formula (XIII) with a base to obtain rasagiline of formula (I).

Racemic 1-aminoindane (X) is commercially available and is used directly in the above described process.

Preferably, the compound of formula (XII) is employed in a molar ratio to racemic 1-aminoindan of formula (X) comprised from about 1.0 to 10.0, preferably from 1.0 to 2.0, more preferably from 1.0 to 1.5.

According to a preferred embodiment the reaction for the preparation of compound of formula (XIV) is carried out in the presence of a base. Examples of base include but are not limited to sodium carbonate, potassium carbonate, sodium bicarbonate, potassium bicarbonate, sodium hydroxide, potassium hydroxide, etc. Preferred bases are sodium carbonate and potassium carbonate, more preferred potassium carbonate.

According to another preferred embodiment, the reaction is carried out in presence of an activating agent, such as an alkali metal halide. Preferred alkali metal halides are sodium iodide and potassium iodide, more preferably potassium iodide.

Preferably, the reaction for the preparation of compound of formula (XIV) is carried out in a polar aprotic solvent, such as acetonitrile, dimethylformamide, dimethylacetamide, *N*-methylpyrrolidone, dimethylsulfoxide, tetrahydrofuran, dioxane or mixtures thereof. Preferred solvent is acetonitrile.

The reaction is carried out at a temperature comprised between about 0° to 100 °C, preferably 20 to 60°C.

Compounds of formula (XIV) are novel compounds and are also object of the present invention. Preferred examples of compound (XIV) are:
*N*-(2-chloroallyl)-2,3-dihydro-1H-inden-1-amine
*N*-(2-bromoallyl)-2,3-dihydro-1H-inden-1-amine

The resolution step of compound (XIV) is achieved with a suitable chiral acid in a suitable solvent. The chiral acid can typically be selected from the group consisting of mandelic acid, tartaric acid, di-p-toluyl tartaric acid, dibenzoyl tartaric acid, and camphor sulphonic acid. Preferably the chiral acid employed in the process of the present invention is (+)-O,O'-Dibenzoyl-D-Tartaric acid.

Preferably, the resolving agent is employed in a molar ratio to compound of formula (XIV) comprised from about 1.0 to 2.0, preferably from 1.0 to 1.5

Suitably the solvent employed is a polar solvent, such as alcohols, for example, methanol, ethanol; or a polar aprotic solvent, such as acetone. A preferred solvent is acetone.

By treating the diastereomeric salt with a basic agent in a suitable solvent, it is possible to liberate the free base, in substantially pure enantiomeric form. Examples of base include but are not limited to sodium carbonate, potassium carbonate, sodium bicarbonate, potassium bicarbonate, sodium hydroxide, potassium hydroxide, ammonium hydroxide and the like. A preferred base employed in the present invention is ammonium hydroxide.

A suitable solvent may be an aromatic hydrocarbon such as toluene; an ester such as ethyl acetate, isopropyl acetate; an ether such as diethyl ether, methyl *tert*-butyl ether (MTBE), and mixtures thereof. Preferably, the solvent is MTBE.

Another object of the present invention is to provide a process for the preparation a (*R*)-aminoindan of formula (IX) 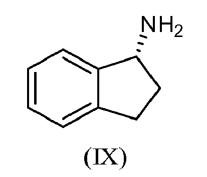 or a pharmaceutically acceptable salt, hydrate, solvate, or polymorph thereof, by hydrolysing a compound of formula (V) or (VI).

According to a preferred embodiment, carbamates of formula (V) or (VI) are treated with an inorganic or organic acid, such as hydrochloric acid, trifluoroacetic acid; or with an inorganic base, such as sodium or potassium hydroxide, piperidine; or with a nucleophilic agent. The nature of the nucleophile can be determined according to methods well known to those skilled in the art, depending on the nature of the group to deprotect. For instance, the nucleophile can be a thiolate, a fluoride anion, an hydride, such as lithium borotriethylhydride, hydrogen.

Preferably, the hydrolysis of the compounds of formula (V) or (VI) to the (*R*)-1-aminoindane (IX), is conducted in a high boiling alcoholic solvent, such as 1- or 2-butanol, followed by extraction and purification of the desired amine with the ordinary methods known to those skilled in the art.

Many of the compounds described in this disclosure, are capable of forming pharmaceutically acceptable salts. These salts include, without limitation, acid addition salts (including diacids). One may prepare a pharmaceutically acceptable acid addition salt by contacting a compound's free base with a sufficient amount of a desired acid to produce a nontoxic salt. If the salt precipitates from solution, it may be isolated by filtration; otherwise, the salt may be recovered by evaporating the solvent.

One may also regenerate the free base by contacting the acid addition salt with a base. Though certain physical properties of the free base and its respective acid addition salt may differ (e.g., solubility, crystal structure, hygroscopicity, etc.), a compound's free base and acid addition salt are otherwise the same for purposes of this disclosure.

It is contemplated that the compounds of the present method can be found or isolated in the form of hydrates or solvates, in different polymorphic forms, i.e., different crystalline forms, which are considered to fall within the scope of the present invention.

In another embodiment, the invention provides the use of compounds of formula (IV), (V) or (VIII), (XIII), (XIV) as defined above, as intermediates in the preparation of (*R*)-1-aminoindanes, preferably Rasagiline.

As mentioned above the compound of the invention has useful therapeutic properties. (*R*)-Rasagiline may be administered per se or, preferably, as a pharmaceutical composition.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
AcOEt (ethyl acetate); DMF (dimethylformamide); NaHCO₃ (sodium hydrogencarbonate); HPLC (high performance liquid chromatography); Rₜ (retention time); TLC (thin layer chromatography).

### Example 1

### (1R,2S,SR)-2-isopropyl-5-methylcyclohexyl (R)-2,3-dihydro-1H-inden-1-ylcarbamate (Va)

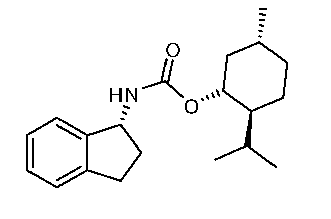

A 100 mL three-necked flask fitted with thermometer and reflux condenser was charged with (±)-2,3-dihydro-1H-indene-1-carboxylic acid (II) (4.00 g, 24.7 mmol), toluene (50 mL), triethylamine (3.6 mL, 25.93 mmol), (1*R*,2*S*,S*R*)-(-)-menthol (4.25 g, 27.2 mmol) and diphenylphosphoryl azide (5.31 mL, 24.7mmol), under nitrogen atmosphere. The resultant clear solution was heated to 80 °C and monitored by TLC, (elution with 10% AcOEt in hexane, visualization: KMnO₄) until complete conversion. After 2 hours, the mixture was cooled, toluene (100 mL) was added and washed with 1N HCl (2 x 50 mL), followed by water and finally with saturated NaHCO₃. The toluene extract was dried over sodium sulfate and concentrated to a residue under reduced pressure. The residue was dissolved in methanol at reflux and slowly cooled to about 50 °C. After the crystallization of the product, the suspension was filtered at the same temperature obtaining 2.3 g of the product (V) with a diastereomeric ratio of 87:13 by HPLC.

The product was recrystallized, as described above obtaining, the diastereomerically pure (V) (1.85 g), by HPLC.

### HPLC conditions:

Discovery Zr-Carbon 15 cm x 4.6 mm x 5 µm; component A: isopropanol, component B: acetonitrile, component C: 5% methanol in water.
Flow 0.5 mL/min, detection at 210 nm, Rₜ 18.7 and 20.6 min.

| Time min | %A | %B | %C |
|---|---|---|---|
| 0 | 50 | 0 | 50 |
| 10 | 100 | 0 | 0 |
| 20 | 50 | 50 | 0 |
| 25 | 50 | 50 | 0 |
| 30 | 50 | 0 | 50 |
| 35 | 50 | 0 | 50 |

¹H NMR (300 MHz, CDCl₃, 298K) 0.81 (d, J=7.0, 3H), 0.91 (m, 6H), 0.95-1.14 (m, 1H), 1.26-1.33 (bt, 1H), 1.45-1.55 (m, 1H), 1.60-1.70 (m, 2H), 1.72-1.82 (m, 1H), 1.85-1.95 (m, 1H), 2.07-2.14 (m, 1H), 2.54-2.65(m, 1H), 2.78.3.00 (m, 2H), 4.60 (td, J=10.7, 4.3, 1H), 4.81 (d, 1H), 5.22 (q, J=8.0, 1H), 7.11-7.28 (m, 4H)
¹³C NMR (75 MHz, CDCl₃, 298K)16.6, 20.9, 22.2, 23.6, 26.4, 30.1, 31.5, 34.4, 41.5, 47.5, 56.4, 74.7, 124.1, 124.8, 126.8, 128.0, 143.2, 143.5, 156.4

### Example 2

### (R)-tert-butyl 2,3-dihydro-1H-inden-1-ylcarbamate (VIa)

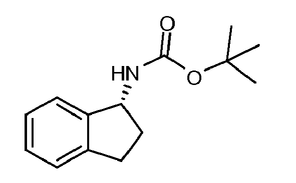

A 100 mL round bottomed flask fitted with reflux condenser, thermometer and a rubber septum was charged, under nitrogen atmosphere, with (1*R*,2*S*,5*R*)-2-isopropyl-5-methylcyclohexyl (*R*)-2,3-dihydro-1*H*-inden-1-ylcarbamate (V) (5.0 g, 15.8 mmol), toluene (50 mL), and tetra *tert*-butyl orthotitanate (6.80 g, 20.0 mmol) was added *via* syringe. The mixture, monitored with TLC analysis (elution with hexane/diethyl ether 1/1, visualization: KMnO₄), was stirred at reflux temperature (110 °C) for 18 hours, until complete conversion. The mixture was then cooled to room temperature, methanol (20 mL) and water (1 mL) were added. The mixture was filtered on Celite^{®} and concentrated to a residue. Menthol was removed by sublimation (0.1 mmHg, 70 °C), obtaining the title compound (VIa) (2.9 g) as a white solid.
Yield: 78%.
¹H NMR (d₆-dmso 300 MHz, 298K): 1.42 (s, 9H), 1.78 (dq, J=12.4, 8.7, 1H), 2.27-2.37 (m, 1H), 2.73 (dt, J=15.8, 8.3, 1H), 2.88 (ddd, J=15.7, 8.8, 3.1, 1H), 3.33 (bs, 1H), 4.97 (q, J=8.2, 1H), 7.11-7.28 (m, 4H)
¹³C NMR (d₆-dmso 75.4 MHz, 298K): 28.8, 30.1, 33.1, 55.7, 78.2, 124.2, 125.0, 126.8, 127.7, 143.1, 145.2, 156.2

### Example 3

### (R)-Isopropyl2,3-dihydro-1H-inden-1-ylcarbamate (VIb)

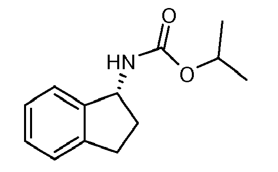

A 100 mL three-necked flask fitted with thermometer and reflux condenser, was charged, under nitrogen atmosphere, with (1*R*,2*S*,5*R*)-2-isopropyl-5-methylcyclohexyl (*R*)-2,3-dihydro-1*H*-inden-1-ylcarbamate (V) (5.0 g, 15.6 mmol), toluene (50 mL) and titanium isopropoxide (10.0 g, 35.2 mmol). The heterogeneous mixture was heated to reflux temperature (120 °C) for 3 hours, until TLC analysis (elution with 5% AcOEt in hexane, visualization: KMnO₄) showed complete conversion. The mixture was then cooled to room temperature and 10% H₂SO₄ was added. After most of the solid was dissolved, the phases were separated and the organic phase was washed again with 10% H₂SO₄. The organic phase was filtered on Celite^{®} and concentrated to a residue. Menthol was removed by sublimation (0.1 mmHg, 70 °C), obtaining the title compound (VIb) (3.1 g) as a white solid.
Yield: 94%.
¹H NMR (300 MHz, CDCl₃, 313K) 1.26 (d, J=6.4, 6H), 1.81 (dq, J=12.8, 8.6, 1H), 2.54-2.63 (m, 1H), 2.78-3.01 (m, 2H), 4.6-4.8 (bs, 1H), 4.97 (sept, J=6.4, 1H), 5.21 (q, J=6.4, 1H), 7.19-7.25 (m, 3H), 7.30-7.32 (m, 1H)
¹³C NMR (75 MHz, CDCl₃, 298K) 22.2, 30.1, 30.3, 61.9, 69.2, 124.3, 125.0, 126.7, 128.0, 155.9

### Example 4

### (R)-isopropyl2,3-dihydro-1H-inden-1-yl(prop-2-ynyl)carbamate (VIIIb)

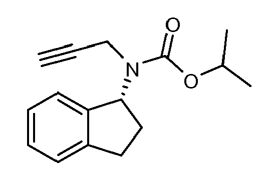

A 50 mL three-necked flask fitted with thermometer, rubber septum and nitrogen inlet was charged with sodium hydride (60% in mineral oil, 632 mg, 15.8 mmol). After 3 washes with hexane (3 x 3mL), DMF (6 mL) was added. The substrate (*R*)-isopropyl 2,3-dihydro-1*H*-inden-1-ylcarbamate (VIb) (2.90 g, 13.2 mmol) was dissolved in THF (20 mL) and added to the suspension of sodium hydride, at 0 °C. The mixture was stirred for 30 minutes at room temperature, then was cooled to 0 °C and a solution of propargyl bromide (80 % in toluene, 1.76 mL, 19.8 mmol) in THF (10 mL) was added over one hour. The mixture was stirred at about 45 °C and monitored by TLC (10% Et₂O in hexane, visualization: KMnO₄) until complete conversion occurred. After 3 hours, water (10 mL) was added, THF was removed under reduced pressure and the residue was taken up in toluene (50 mL). The aqueous phase was washed with water, brine and then dried over sodium sulfate. The residue was purified by flash chromatography with gradient elution hexanes/Et₂O 90/10 to 80/20, obtaining the title compound (VIIIb) (2.6 g), as a yellow oil.
Yield: 76%.
¹H NMR (300 MHz, CDCl₃, 313K) 1.0-1.5 (bs, 6H), 2.07 (s, 1H), 2.08-2.34 (m, 1H), 2.34-2.39 (m,1H), 2.74-2.80 (m, 1H), 2.94-3.10 (m, 1H), 3.30-3.60 (bs, 1H), 3.8-4.4 (bs, 1H), 4.96 (sept, J=6.1, 1H), 5.3-6.0 (bs, 1H), 7.00-7.30 (m, 4H)
¹³C NMR (75 MHz, CDCl₃, 313K) 22.2, 30.1, 30.2, 30.3, 61.9, 69.1, 70.5, 81.3, 124.3, 124.9, 125.2 126.6, 141.2, 143.7, 155.8

### Example 5

### (R)-Tert-butyl 2,3-dihydro-1H-inden-1-yl(prop-2-ynyl)carbamate (VIIIa)

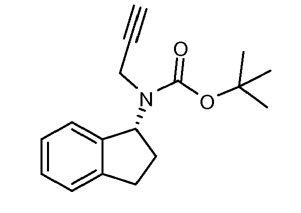

A 50 mL two-necked flask was charged with sodium hydride (60% in mineral oil, 98 mg, 2.35 mmol), under nitrogen atmosphere, and was washed twice with 2 mL of dry hexane. (*R*)-*Tert*-butyl 2,3-dihydro-1*H*-inden-1-ylcarbamate (VIa) (500 mg, 2.14 mmol), dissolved in anhydrous DMF (5 mL), was added via syringe. After stirring for 15 minutes at room temperature, propargyl bromide (80% solution in toluene, 286 µL, 2.57 mmol) was added and the mixture was stirred for 4 hours at room temperature, then quenched with water (10 mL) and extracted with toluene (20 mL). The organic layer was washed with water (2 x 5 mL), brine (5 mL) and dried over sodium sulfate. After removal of the solvent under reduced pressure the residue was purified by flash chromatography, eluting with 5% AcOEt in hexane, obtaining the title compound (VIIIa), as a yellow oil.
Yield: 77%.
¹H NMR (CDCl₃, 300 MHz, 298K): 1.2-1.7 (bs, 9H), 2.10-2.15 (bs, 1H), 2.15.2.30 (bs, 1H), 2.40-2.50 (m, 1H), 2.80-2.90 (m, 1H), 2.95-3.1 (m, 1H), 3.3-4.3 (bm, 2H), 5.4-5.8 (bm, 1H), 7.10-7.32 (m, 4H)
¹³C NMR (CDCl₃, 75.4 MHz, 298K): 27.8, 28.4, 30.2, 30.9, 62.0, 70.0, 80.4, 124.3, 125.0, 126.6, 127.8, 138.9, 143.2, 155.4

### Example 6

### (R)-N-prop-2-ynyl-2,3-dihydro-1H-inden-1-amine (Rasagiline base)

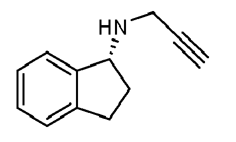

A 50 two-necked flask, was charged, under nitrogen atmosphere, with (*R*)-tert-butyl 2,3-dihydro-1*H*-inden-1-yl(prop-2-ynyl)carbamate (VIIIa) (300 mg, 1.10 mmol), dissolved in dioxane (11 mL). 37% HCl in water (0.5 mL) was added. After stirring for 30 minutes at room temperature, the reaction mixture was quenched with a saturated solution of NaHCO₃ in water (10 mL). Dioxane was removed under reduced pressure and the aqueous layer was extracted with diethyl ether (3 x 20 mL). The organic phase was dried over sodium sulfate and concentrated to a residue, obtaining the title compound (177 mg), as a brown oil.
Yield: 95%.
¹H NMR (CDCl₃, 300 MHz, 298K): 1.69 (bs, 1H), 1.82-1.91 (m, 1H), 2.28 (t, J=2.5, 1H), 2.38-2.41 (m, 1H), 2.83 (ddd, J=15.8, 8.2, 6.3, 1H), 3.04 (ddd, J=15.9, 8.3, 5.8, 1H), 3.50 (dd, J=16.8, 2.5, 1H), 3.52 (dd, J=16.9, 2.1, 1H), 4.42(t, J=6, 1H), 7.16-7.27 (m, 3H), 7.31-7.38 (m, 1H)
¹³C NMR (CDCl₃, 75.4 MHz, 298K): 30.6, 33.4, 36.2, 62.0, 71.6, 82.7, 124.3, 125.0, 126.4, 127,7, 143.9, 144.6

### Example 7

### (R)-1-Aminoindane (IX)

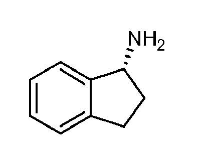

A 50 mL round bottomed flask fitted with A 100 mL three-necked flask fitted with thermometer, reflux condenser and under nitrogen atmosphere was charged with (1*R*,2*S*,5*R*)-2-isopropyl-5-methylcyclohexyl (*R*)-2,3-dihydro-1*H*-inden-1-ylcarbamate (V) (10.0 g, 31.7 mmol), 1-butanol (10 mL) and potassium hydroxide (8.35 g, 126.8 mmol). The mixture was heated to reflux and the reaction was monitored by TLC (elution with hexane-diethyl ether 1:1, visualization with KMnO₄) until complete conversion, then cooled to room temperature, added of toluene (50 mL) and water (30 mL). The organic phase was washed twice with water (10 mL), then twice with 1N HCl (20 mL). The acidic organic phase was washed with diethyl ether (10 mL), then the pH was corrected to 10 by addition of 30% sodium hydroxide and the product was extracted into diethyl ether. The extract was dried over sodium sulfate and concentrated to dryness affording the title product (3.4 g, 81%) as a dark green oil, spectroscopically identical to an authentic sample.

### Example 8

### (R)-N-(2-chloroallyl)-2,3-dihydro-1H-inden-1-amine (crude) (XIIIa)

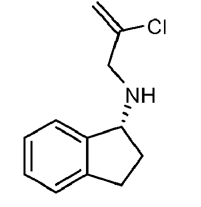

A 100 mL round bottomed flask was charged under nitrogen with (*R*)-1-aminoindane (IX) (5.43 g, 40.8 mmol), acetonitrile (50 mL), potassium carbonate (8.44 g, 61.2 mmol), potassium iodide (317 mg, 1.9 mmol) and 2,3-dichloro-1-propene (XII) (3.8 mL, 41.2 mmol). The resulting mixture was heated to reflux under nitrogen for 4-6 hours monitoring the reaction by GLC and stopped when the 1-aminoindane was less than 5% in area to the desired product.
After cooling to room temperature water (50 mL) was added and the product is extracted with toluene (50 mL). The aqueous phase was extracted with toluene (2 x 25 mL) and the combined organic layers were dried over sodium sulfate, filtrated and the solution concentrated to a residue, which contained 4.8% aminoindane and 8.1% dialkylated product by gas-chromatography.

### Example 9

### (R)-N-(2-chloroallyl)-2,3-dihydro-1H-inden-1-amine oxalate (XIIIb)

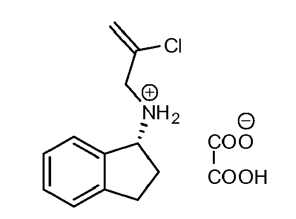

The product from the reaction above was purified by dissolution in methanol (40 ml) and portionwise addition of oxalic acid (3.67 g, 40.8 mmol). The slurry of the oxalate salt was refluxed for 30 minutes, then cooled to room temperature, filtered and washed with methanol to afford 10.1 g of product as a white solid, m.p. 198.3 - 198.5 °C showing the x-ray diffraction pattern reported below (Figure 1).

### Example 10

### (R)-N-(2-chloroallyl)-2,3-dihydro-1H-inden-1-amine (pure) (XIIIa)

The pure free base was obtained by suspending the oxalate salt from the example above in MTBE (70 mL) and adding 15% ammonium hydroxide (30 mL), separating the phases and drying the organic phase on sodium sulfate. The solvent was evaporated under reduced pressure and the free base is obtained as an almost colourless oil (6.8 g, 80% yield), 99.6% pure by GC with dialkylated product < 0.05%.
GC Method: Agilent 6850 gas-chromatograph, carried gas: He, split ratio 1:10, inlet T=250 °C, detector: 300 °C, HP-1 column, 30 m, i.d. 0.320 mm, film thickness 0.25 µm. Ramp: 50 °C hold 1 min, then 50 to 110 °C at 10 °C/min, then 110 to 230 °C at 40 °C/min, then 230 °C hold 15 min.
Retention times: 1-aminoindane 8.84 min, *N*-(2-chloroallyl) derivative 11.35 min, *N*,*N*-bis(2-chloroallyl) derivative 13.2 min.
¹H NMR (CDCl₃, 300 MHz, 298K) 1.75 (bs, 1H,), 1.78-1.87 (m, 1H), 2.30-2.38 (m,1H), 2.75-2.86 (m, 1H), 2.97-3.08 (m, 1H), 3.50 (s, 2H), 4.25 (t, J=6.4, 1H), 5.34 (d, J=1.2, 1H), 5.46 (d, J=1.2, 1H), 7.12-7.25 (m, 3H), 7.30-7.38 (m, 1H)
¹³C NMR (CDCl₃, 75 MHz, 298K) 30.5, 33.5, 53.5, 61.8, 113.2, 124.1, 124.9, 126.4, 127.7, 141.5, 143.7, 144.9

### Example 11

### (±)-N-(2-chloroallyl)-2,3-dihydro-1H-inden-1-amine (XIVa)

The product above was obtained as described for example 10 employing racemic 1-aminoindane (X).

### Example 12

### (R)-N-prop-2-ynyl-2,3-dihydro-1H-inden-1-amine (Rasagiline base) (I)

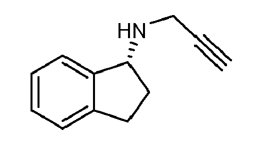

### Method 1

A 100 mL round bottomed flask was charged with (*R*)-N-(2-chloroallyl)-2,3-dihydro-1H-inden-1-amine (XIII) (3.0 g, 14.4 mmol), dimethylsulfoxide (30 mL) and powdered KOH (85%, 2.85 g, 43.2 mmol) and stirred at 25° C for 36 hours, then the mixture was diluted with toluene (50 mL) and washed three times with water (30 mL).
The organic layer was dried over sodium sulfate and the residue was purified by flash chromatography on silica gel eluting with 5% MeOH in AcOEt, affording 1.8 g of the title compound as a light green oil (74% yield).

### Method 2

A 100 mL round bottomed flask under nitrogen was charged with (*R*)-N-(2-chloroallyl)-2,3-dihydro-1H-inden-1-amine (XIII) (3.0 g, 14.4 mmol), dimethylacetamide (15 mL) and was cooled to 0 °C. To the cooled solution was added sodium tert-butoxide (1.66 g, 17.3 mmol) divided into 4 portions every 30 minutes. After further 40 minutes at 0-5 °C the mixture was diluted with toluene (50 mL) and washed three times with water (15 mL). The organic layer was dried over sodium sulfate and the residue was purified by flash chromatography on silica gel eluting with 5% MeOH in AcOEt, affording 1.99 g of the title compound as a light green oil (82% yield).
The mesylate salt was obtained from isopropanol, .[α]_{D}= +25.2 ° (c=1, EtOH).
¹H NMR (CDCl₃, 300 MHz, 298K): 1.69 (bs, 1H), 1.82-1.91 (m, 1H), 2.28 (t, J=2.5, 1H), 2.38-2.41 (m, 1H), 2.83 (ddd, J=15.8, 8.2, 6.3, 1H), 3.04 (ddd, J=15.9, 8.3, 5.8, 1H), 3.50 (dd, J=16.8, 2.5, 1H), 3.52 (dd, J=16.9, 2.1, 1H), 4.42(t, J=6, 1H), 7.16-7.27 (m, 3H), 7.31-7.38 (m, 1H)
¹³C NMR (CDCl₃, 75.4 MHz, 298K): 30.6, 33.4, 36.2, 62.0, 71.6, 82.7, 124.3, 125.0, 126.4, 127,7, 143.9, 144.6

### Example 13

### Resolution of (±)-N-(2-chloroallyl)-2,3-dihydro-1H-mden-1-amme (XIVa)

A 50 mL round bottomed flask was charged with (±)-*N*-(2-chloroallyl)-2,3-dihydro-1H-inden-1-amine (XIV) (2.4 g, 11.55 mmol), acetone (12 mL) and (+)-O,O'-Dibenzoyl-D-tartaric acid (4.14 g, 11.55 mmol). The slurry was heated to reflux for 30 minutes, then cooled to room temperature in two hour. The product was filtered and washed with acetone (2 x 3 mL) at room temperature. The product was dried under reduced pressure at 50 °C for four hours, obtaining 2.84 g of product.

The salt was suspended in water (10 mL) and MTBE (7 mL) and 10% aqueous ammonia was added until pH=9. After phase separation the organic layer was dried over sodium sulfate and concentrated under reduced pressure 1.01 g of the title product. The optical purity was determined by transformation into Rasagiline base as described in Example 5 and converting the free base into the mesylate salt with methanesulfonic acid in isopropanol. [α]_{D}= +5.2 ° (c=1, EtOH)

The enantiomeric purity was upgraded by repeating the crystallization step of the dibenzoyl tartrate from acetone.

## Claims

1. A process for the preparation of a compound of formula (V), 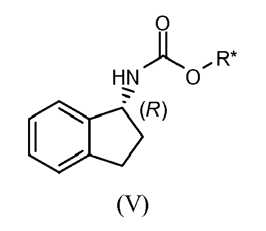 with the (*R*)-configuration at the 1-position of the indane ring; wherein R* is a chiral moiety having at least one asymmetric center;
the process comprising:
a) reacting a compound of formula (II), 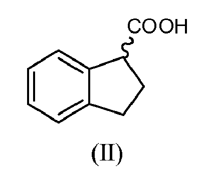 with a reagent capable of transforming a compound of formula (II) into an isocyanate of formula (III), 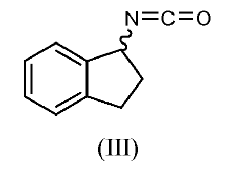 and
b) reacting compound of formula (III) with a chiral alcohol R*-OH, wherein R* is a chiral moiety having at least one asymmetric center, to obtain a carbamate of formula (IV), as a mixture of diastereoisomers, 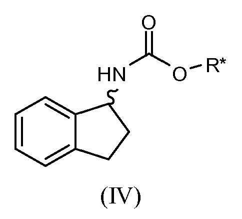 wherein R* is as defined above;
c) resolving the mixture of diastereoisomers of carbamates of formula (IV) to obtain a diastereoisomer of formula (V).

2. The process according to claim 1, wherein R* is selected from the group consisting of (-)-menthyl, (+)-menthyl, (-)-8-phenylmenthyl, (+)-8-phenylmenthyl, (-)-neomenthyl, (+)-neomenthyl and (R) and (S)-1-phenylethyl.

3. A process for the preparation of Rasagiline (I) 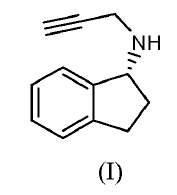 or a pharmaceutically acceptable salt, hydrate, solvate, or polymorph thereof, said process comprising carrying out the preparation process of compound V according to any of the claims 1 or 2.

4. A process for the preparation of Rasagiline (I), or a pharmaceutically acceptable salt, hydrate, solvate, or polymorph thereof, said process comprising:
a) converting the compound of formula (V), 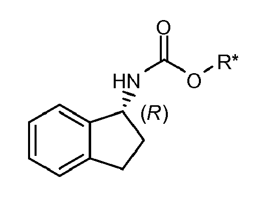 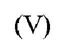 wherein R* is a chiral moiety having at least one asymmetric center; to an (*R*)-aminoindan of formula (IX) 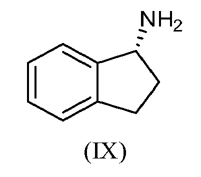 and;
b) reacting (R)-aminoindan of formula (IX) with a dihalopropene compound of formula (XII): 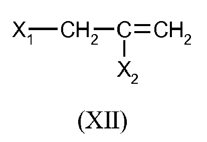 wherein X₁ and X₂ independently represent a chlorine, bromide or iodine, to obtain a haloallyl derivative of formula (XIII): 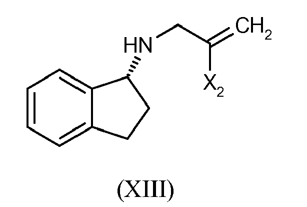 or a pharmaceutically acceptable salt, hydrate, solvate, or polymorph thereof wherein X₂ is as defined above; and
c) dehydrohalogenating compound of formula (XIII) with a base to obtain rasagiline of formula (I).

5. The process according to claim 4, wherein R* is selected from the group consisting of (-)-menthyl, (+)-menthyl, (-)-8-phenylmenthyl, (+)-8-phenylmenthyl, (-)-neomenthyl, (+)-neomenthyl or (*R*) or (*S*)-1-phenylethyl

6. A process for the preparation of Rasagiline of formula (I), 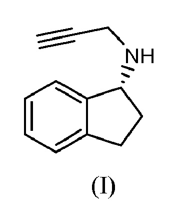 or a pharmaceutically acceptable salt, hydrate, solvate, or polymorph thereof, said process comprising:
a) transterification of carbamate of formula (V) 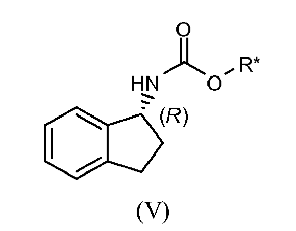 wherein R* is a chiral moiety having at least one asymmetric center;
into a carbamate of formula (VI) 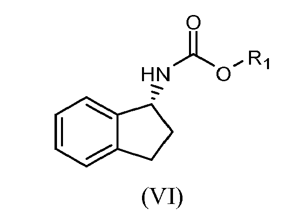 wherein R₁ is a C₁-C₆ alkyl group optionally substituted with halogens, an aryl optionally substituted or a trialkyl silyl group; and
b) reacting a compound of formula (VI) with a compound of formula (VII), 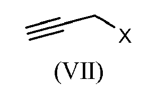 wherein X is a leaving group; to obtain a compound of formula (VIII) 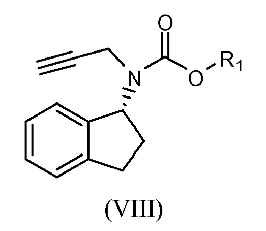 wherein R₁ is as defined above; and
c) deprotecting compound of formula (VIII) to give the Rasagiline (I).

7. The process according to claim 6, wherein R* is selected from the group consisting of (-)-menthyl, (+)-menthyl, (-)-8-phenylmenthyl, (+)-8-phenylmenthyl, (-)-neomenthyl, (+)-neomenthyl or (*R*) or (*S*)-1-phenylethyl.

8. A process for the preparation of (*R*) amino indane of formula (IX), 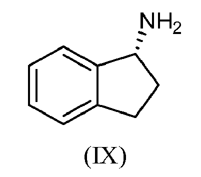 or a pharmaceutically acceptable salt, hydrate, solvate, or polymorph thereof, by hydrolyzing a compound of formula (V) or (VI) as defined in claim 6.

9. A compound of formula (IV), or a pharmaceutically acceptable salt, hydrate, solvate, or polymorph thereof 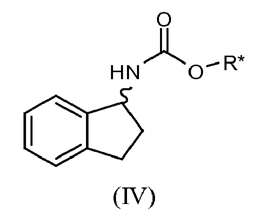 wherein R* is selected from the group consisting of (-)-menthyl, (+)-menthyl, (-)-8-phenylmenthyl, (+)-8-phenylmenthyl, (-)-neomenthyl, (+)-neomenthyl and (*R*) and (*S*)-1-phenylethanol, and the wavy bond refers to both (*R*) and (*S*) configuration at the C-N bond of the indan ring.

10. A compound of formula (V), or a pharmaceutically acceptable salt, hydrate, solvate, or polymorph thereof 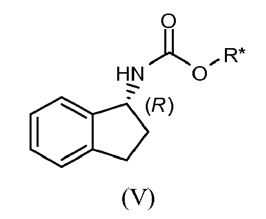 wherein R* is selected from the group consisting of (-)-menthyl, (+)-menthyl, (-)-8-phenylmenthyl, (+)-8-phenylmenthyl, (-)-neomenthyl, (+)-neomenthyl and (*R*) and (*S*)-1-phenylethanol.

11. The compound according to any one of the claims 9-10 selected from:
(1*R*,2*S*,5*R*)-2-isopropyl-5-methylcyclohexyl (*R*)-2,3-dihydro-1H-inden-1-ylcarbamate;
(1*R*,2*S*,5*R*)-2-isopropyl-5-methylcyclohexyl (*S*)-2,3-dihydro-1H-inden-1-ylcarbamate;
(1*S*,2*R*,5*S*)-2-isopropyl-5-methylcyclohexyl (*R*)-2,3-dihydro-1H-inden-1-ylcarbamate; and
(1*S*,2*R*,5*S*)-2-isopropyl-5-methylcyclohexyl (*S*)-2,3-dihydro-1H-inden-1-ylcarbamate.

12. A compound of formula (VI), or a pharmaceutically acceptable salt, hydrate, solvate, or polymorph thereof 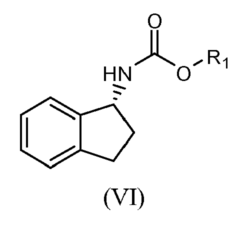 wherein R₁ is a C₁-C₆ alkyl group optionally substituted with halogens, an aryl optionally substituted or a trialkyl silyl group; with the proviso that R₁ is not a t-butyl group.

13. A compound of formula (VIII), or a pharmaceutically acceptable salt, hydrate, solvate, or polymorph thereof 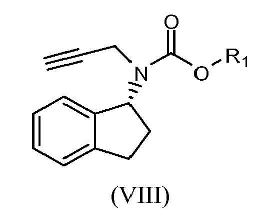 wherein R₁ is a C₁-C₆ alkyl group optionally substituted with halogens, an aryl optionally substituted or a trialkyl silyl group.

14. A compound of formula (XIII), or a pharmaceutically acceptable salt, hydrate, solvate, or polymorph thereof 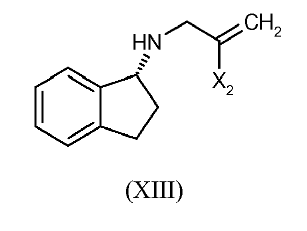 wherein X₂ represents chlorine, bromine or iodine

15. Use of compounds of formula (IV), (V), (VI), (VIII), or (XIII) as defined in any one of claims 9-14, in the preparation of rasagiline.
